# EUROPÄISCHE PATENTSCHRIFT

(11) **EP 1 212 272 B1**
(45) Veröffentlichungstag und Bekanntmachung des Hinweises auf die Patenterteilung: **10.12.2003**
(21) Anmeldenummer: 00964140.8
(22) Anmeldetag: 08.09.2000
(51) Int. Cl.: C07C 45/74, C07C 47/225, C07C 29/141, C07C 33/12

(54) **VERFAHREN ZUR HERSTELLUNG VON ALKYL-SUBSTITUIERTEN BUTENOLEN**
METHOD FOR PRODUCING ALKYL-SUBSTITUTED BUTENOLS
PROCEDE DE PREPARATION DE BUTENOLS A SUBSTITUTION ALKYLE

(30) Priorität: 17.09.1999 DE 19944524
(43) Veröffentlichungstag der Anmeldung: 12.06.2002
(73) Patentinhaber: Kao Corporation, Chuo-Ku Tokyo 103-8210 (JP)
(72) Erfinder: HECK, Stephan, 50259 Pulheim (DE); FRIESENHAGEN, Lothar, 40589 Düsseldorf (DE); KLEIN, Norbert, 40822 Mettmann (DE); MARKERT, Thomas, 40789 Monheim (DE); PELZER, Gerrit, 40589 Düsseldorf (DE); SCHNEIDER, Markus, 47279 Duisburg (DE)
(74) Vertreter: Wolf, Matthias
(86) Internationale Anmeldenummer: EP0008772
(87) Internationale Veröffentlichungsnummer: WO01021568

(56) Entgegenhaltungen:
- EP-A- 0 829 463
- DE-A- 19 520 103

## Beschreibung

### Gebiet der Erfindung

Die Erfindung betrifft ein Verfahren zur Herstellung von Alkyl-substituierten Butenolen durch Reduktion der entsprechenden Aldehyd-Vorstufe in Gegenwart von Kupfer-Zink-Kontakten, wobei man das Verfahren in kontinuierlicher und isothermer Fahrweise und in einem engen Temperaturbereich durchführt.

### Stand der Technik

Viele natürliche Riechstoffe stehen, gemessen am Bedarf, in völlig unzureichender Menge zur Verfügung. Aus parfümistischer Sicht besonders geschätzt und wertvoll ist das Sandelholzöl. Es wird durch Wasserdampfdestillation aus dem Kernholz des Sandelbaumes gewonnen, eines tropischen Halbparasiten, der in Indien und Malaysia vorkommt. Kernholz erscheint nach etwa zehn Jahren und beginnt erst bei zwanzigjährigen Bäumen, sich rascher auszubilden. Voll ausgewachsene Bäume werden im Alter von 30 bis 60 Jahren ausgerodet, da die Wurzeln besonders reich an wohlriechendem Kernholz sind [vergl. **E.T.Morris, Dragoco Report 1983 (30), 40].** Es ist daher verständlich, daß die Riechstoff-Forschung ständig bemüht ist, geeignete Substitute für natürliches Sandelholzöl zu entwickeln.

Die Schwerpunkte bei der Entwicklung geeigneter Substitute für natürliches Sandelholzöl hat R.E.Naipawer in einem Review skizziert [in: **B.M.Lawrence, B.D.Mookherjee, B.J.Willis (Hrsg.): "Flavors and Fragrances: A World Perspective"; Elsevier Publishers, Amsterdam 1988].** In diesem Review ist unter anderem dargestellt, daß seit Mitte der 70-er Jahre Campholenylderivate eine wichtige Rolle unter den synthetisch hergestellten Riechstoffen mit Sandelduft darstellen. Eine wesentliche Rolle bei diesem Zugang zu synthetischen Sandelriechstoffen hat dabei die Tatsache gespielt, daß Campholenaldehyd, der den referierten Verbindungen zugrundeliegende Synthesebaustein, leicht aus alpha-Pinen zugänglich ist.

2-Alkyl-4-(2,2,3-trimethylcyclopent-3-enyl)-but-2-en-1-ole (A), fortan als **Sandelole** bezeichnet, sind begehrte Riechstoffe mit ausgeprägtem Sandelduft.

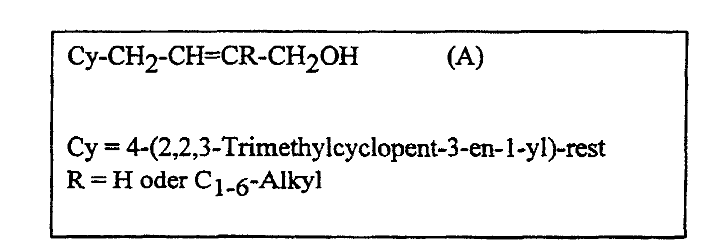

**JP-A2-55/036423** (zitiert nach Chem. Abstr. 93/094886p) beschreibt ein Verfahren zur Herstellung eines solchen Sandelols. Demnach wird alpha-Campholenaldehyd (B) in Gegenwart von Natriumhydroxid als basischem Katalysator mit Propionaldehyd (CH₃-CH₂-CHO) umgesetzt.

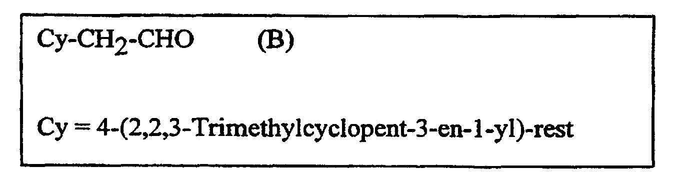

Der bei dieser gemischten Aldolkondensation entstehende ungesättigte Aldehyd (C) wurde in einer Ausbeute von 73,5% isoliert.

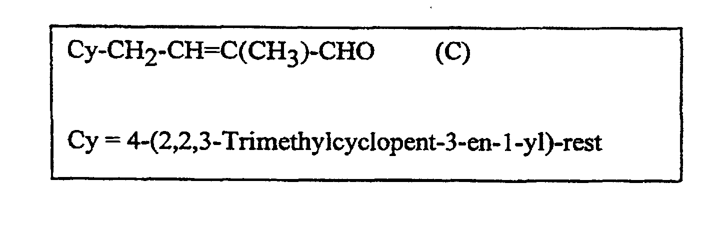

In einem weiteren Schritt wurde schließlich dieser ungesättigte Aldehyd (C) mit Al(OCH(CH₃)₂]₃ zum entsprechenden Sandelol (A) reduziert. Die dabei erzielte Ausbeute wird mit 85% angegeben.

**DE-A-195 20 103** beschreibt ein Verfahren zur Herstellung von Alkyl-substituierten Butenolen, wobei in einem ersten Schritt ungesättigte Aldehyde auf dem Wege einer Aldolkondensation hergestellt werden und diese Produkte anschließend in Gegenwart eines Kupfer-Zink-Kontaktes, der gegebenenfalls calciniert sein kann, reduziert werden. Im Beispiel 3 dieser Anmeldung (Seite 5, Zeilen 5 bis 26), die die zweite Stufe des Verfahrens (Reduktionsschritt) beschreibt, wird eine diskontinuierliche Arbeitsweise (Batchverfahren) und eine Reaktionstemperatur von 160 °C offenbart.

Über eine kontinuierliche Arbeitsweise in Verbindung mit einem äußerst engen und relativ niedrigen Temperaturbereich ist in der DE-A-195 20 103 weder etwas offenbart noch nahegelegt. Auch über eine isotherme Arbeitsweise ist in der DE-A-195 20 103 nichts ausgesagt.

### Beschreibung der Erfindung

Die aus dem Stand der Technik bekannten Verfahren zur Herstellung von Sandelolen (A) vermögen im Hinblick auf Ausbeute und Wirtschaftlichkeit nicht vollständig zu befriedigen. Es bestand daher Bedarf nach einem verbesserten Verfahren zur Herstellung der Verbindungen (A) sowie analoger Verbindungen, bei denen der Rest "Cy" durch einen anderen gesättigten oder olefinisch ungesättigten, gegebenenfalls substituierten Alkyl- oder Cycloalkylrest ersetzt ist. Dabei sollte insbesondere sichergestellt sein, daß eine unspezifische Bildung zahlreicher Nebenprodukte weitgehend vermieden wird. Hierbei wird unter Nebenprodukten verstanden, daß es sich um im parfümistischen Sinne wertlose oder gar störende Produkte handelt.

Es wurde nun überraschend gefunden, daß sich Alkyl-substituierte Butenole der allgemeinen Formel (I),

R¹-CH₂-CH=CR²-CH₂OH (I)

worin R¹ eine gesättigte oder olefinisch ungesättigte Alkyl- oder Cycloalkylgruppe mit 4 bis 16 C-Atomen ist, die gegebenenfalls durch einen Alkyl-, Cycloalkyl-, Aryl- oder Alkarylrest substituiert sein kann - mit der Maßgabe, daß dieser Substituent maximal 12 C-Atome aufweist - und R² Wasserstoff oder eine Alkylgruppe mit 1 bis 6 C-Atomen bedeuten, in hohen Ausbeuten herstellen lassen, wenn man die Aldolkondensation von Aldehyden der Formel (II)

R¹-CH₂-CHO (II)

worin R¹ dieselbe Bedeutung hat wie in Formel (I), und dem entsprechenden niedrigen Aldehyd in einem inerten organischen Lösungsmittel durchführt und die dabei erhaltenen ungesättigten Aldehyde in Gegenwart eines gegebenenfalls calcinierten Kupfer-Zink-Kontaktes reduziert, wobei man das Verfahren in kontinuierlicher und isothermer Fahrweise und in einem engen Temperaturbereich durchführt.

Gegenstand der vorliegenden Erfindung ist daher ein Verfahren zur Herstellung von Alkyl-substituierten Butenolen der allgemeinen Formel (I),

**R**^{**1**}**-CH**_{**2**}**-CH=CR**^{**2**}**-CH**_{**2**}**OH (I)**

worin R¹ eine gesättigte oder olefinisch ungesättigte Alkyl- oder Cycloalkylgruppe mit 4 bis 16 C-Atomen ist, die gegebenenfalls durch einen Alkyl-, Cycloalkyl-, Aryl- oder Alkarylrest substituiert sein kann - mit der Maßgabe, daß dieser Substituent maximal 12 C-Atome aufweist - und R² Wasserstoff oder eine Alkylgruppe mit 1 bis 6 C-Atomen bedeuten, durch Umsetzung von Aldehyden der Formel (II)

**R**^{**1**}**-CH**_{**2**}**-CHO (II)**

worin R¹ dieselbe Bedeutung hat wie in Formel (I), mit den entsprechenden niedrigen Aldehyden und anschließende Reduktion der dabei erhaltenen ungesättigten Aldehyde, wobei man
i) die Aldolkondensation in einem inerten organischen Lösungsmittel durchführt und
ii) die Reduktion der ungesättigten Aldehyde in Gegenwart eines gegebenenfalls calcinierten Kupfer-Zink-Kontaktes durchführt
mit der Maßgabe, daß man die Reduktion in Stufe ii) in kontinuierlicher und isothermer Fahrweise und bei Temperaturen im Bereich von 45 - 60 °C und bei einem Wasserstoffdruck im Bereich von 1 bis 300 bar kontinuierlich durchführt.
Das erfindungsgemäße Verfahren hat gegenüber dem herkömmlichen Stand der Technik den Vorteil, daß Zwischen- und Wertprodukte in hoher Reinheit und in nahezu quantitativer Ausbeute erhalten werden. Es hat insbesondere den Vorteil, daß das bei der Hydrierung entstehende Wertprodukt (I) in sehr hoher chemischer Reinheit und Selektivität entsteht. Von besonderer Bedeutung ist in diesem Zusammenhang, daß unerwünschte Nebenprodukte, beispielsweise von (I) abgeleitete Verbindungen, deren C=C-Doppelbindungen ganz oder teilweise hydriert sind, sowie nicht umgesetzter (aus der Aldolkondensation stammender) Aldehyd im Vergleich zu Fahrweisen, bei denen die erfindungsgemäß einzuhaltenden Merkmale nicht gegeben sind, in deutlich verringerter Menge entstehen. Dies ist insbesondere dann von Vorteil, wenn das bei der Hydrierung erhaltene Wertprodukt (I) als Rohstoff zur Herstellung von Riechstoffen eingesetzt wird.

### Zu Schritt i)

Als inerte organische Lösungsmittel für Stufe i) eignen sich insbesondere unpolare, die mit Wasser ein Azeotrop bilden. Beispiele für geeignete Lösungsmittel sind Toluol, Xylol, Benzol, Cyclohexan und Methylcyclohexan.

In einer speziellen Ausführungsform der Erfindung setzt man zur Katalyse der Aldolkondensation Ammoniumsalze einer organischen Säure ein.

Propionaldehyd wird vorzugsweise in 2,5- bis 10-molarem Überschuß - bezogen auf den Aldehyd (II) - eingesetzt. Insbesondere setzt man Propionaldehyd in 2,5 bis 3,5-molarem Überschuß ein.

Wie bereits gesagt, wird im Zuge des erfindungsgemäßen Verfahrens die Aldolkondensation vorzugsweise in Gegenwart eines Ammoniumsalzes einer organischen Säure durchgeführt. Die Art der Säure ist dabei an sich nicht kritisch. Ebenso wenig spielt es eine Rolle ob das Ammoniumsalz als solches eingesetzt wird oder ob es während der Reaktion - etwa aus einem Amin und einer organischen Säure - in situ gebildet wird. Beispiele für geeignete Ammoniumsalze sind: Benzyltrimethylammoniumhydroxid, Piperidinylacetat, Pyrrolidiniumacetat, Ammoniumacetat, Dimethylammoniumpyridinylacetat, Morpholinacetat, Lewatit 11600 (mit Essigsäure aktiviert), Piperidinylformiat, N,N-Tetraacetylethylendiamin, N,N-Diacetylethylendiamin, Dibutylammoniumacetat und Piperidinylpropionat. Die Konzentration des Katalysators liegt vorzugsweise im Bereich von 0,001 bis 20 Mol-%, insbesondere 0,5 bis 10 Mol-% - bezogen auf den eingesetzten Aldehyd (II).

In einer weiteren bevorzugten Ausführungsform der vorliegenden Erfindung bedeutet R¹ in der allgemeinen Formel (I) einen 4-(2,2,3-Trimethylcyclopent-3-enyl)-Rest.

### Zu Schritt ii)

Das erfindungsgemäße Verfahren wird bei Temperaturen im Bereich von 45 bis 60 °C durchgeführt. Dabei ist es besonders bevorzugt, eine Reaktionstemperatur im Bereich von 50 bis 55 °C einzustellen.

Das erfindungsgemäße Verfahren zeichnet sich unter anderem dadurch aus, daß es in Schritt ii) sowohl in kontinuierlicher Weise als auch isotherm durchgeführt wird.

Die kontinuierliche Durchführung des erfindungsgemäßen Verfahrens in Schritt ii), die vorzugsweise in einem Festbettreaktor durchgeführt wird, stellt in ganz besonderem Maße sicher, daß der Anteil an Nebenprodukten gering ist. Der Grund dafür ist einerseits darin zu sehen, daß es bei dieser Arbeitsweise über die Steuerung des Volumenstroms von Aldehyd bzw. Aldehyd und Solvens, leicht möglich ist, die Reaktionszeit gering zu halten; andererseits garantiert die hier angewandte heterogene Katalyse, daß das Reaktionsprodukt nicht mit nennenswerten Mengen des Katalysators belastet ist.

Unter einer isothermen Fahrweise wird verstanden, daß im kontinuierlich betriebenen Reaktor keine wesentlichen Temperaturgradienten auftreten und daß insbesondere keine Temperaturspitzen auftreten. Im Gegensatz dazu wäre eine nicht-isotherme Reaktionsweise dadurch gekennzeichnet, daß im Reaktor deutliche Temperaturgradienten bzw. Temperaturspitzen auftreten. Eine nicht-isotherme Fahrweise ist üblicherweise bei der Batch-Hydrierung, also einer Hydrierung in einem Autoklaven gegeben. Eine nicht-isotherme Fahrweise ist bei kontinuierlichem Betrieb dann gegeben, wenn keine speziellen Vorkehrungen getroffen werden, um die Exothermie der Reaktion gezielt zu kontrollieren.

Die vorliegende Erfindung sieht zur Realisierung einer isothermen Fahrweise insbesondere vor, die Temperaturkontrolle durch eine äußere Mantelheizung mit beispielsweise Thermalöl zu gewährleisten und/oder die Durchflußgeschwindigkeit entsprechend hoch einzustellen.

Insbesondere stellt man die Durchflußgeschwindigkeit in Schritt ii) auf Werte im Bereich von 0,5 bis 1,5 m³/h und insbesondere 0,8 bis 1,2 m³/h ein.

Eine bevorzugte Ausführungsform der Erfindung ist dadurch charakterisiert, daß man den Kupfer-Zink-Katalysator in stückiger Form einsetzt. Der Katalysator, der in der Reaktionszone des Festbettreaktors eingesetzt wird, liegt dabei in Form fester Partikel vor (heterogener Katalysator). Die Partikel können dabei die unterschiedlichsten Größen und Formen aufweisen, z. B. Tabletten, Klumpen, Zylinder, Stangen, Ringe. Die Größe der Partikel ist an sich nicht kritisch. Üblicherweise wird sie jedoch den jeweils vorliegenden Reaktordimensionen derart angepaßt, daß flüssige Phase und Trägergas die Reaktionszone ungehindert passieren können und kein unerwünschter Druckabfall in diesem Bereich auftritt. Typische geeignete Partikelgrößen reichen von einem mittleren Durchmesser von ca. 1 Millimeter bis ca. 10 Millimeter, obwohl auch größere bzw. kleinere Teilchengrößen nicht ausgeschlossen sind.
Eine typische Laborapparatur zur Durchführung des erfindungsgemäßen Verfahrens umfaßt einen Festbettreaktor aus einem Doppelmantelrohr. Das innere Rohr enthält den heterogenen Hydrierungskatalysator und dient als Reaktionszone; der Mantelzwischenraum dient der Beheizung mit einem flüssigen Medium. Der Aldehyd bzw. die Mischung von Aldehyd und Solvens läßt sich kontinuierlich mittels einer regelbaren Kolbenmembranpumpe über beheizbare Zuleitungen zuführen. Die gebildeten Reaktionsprodukte lassen sich nach dem Verlassen des Reaktors über ein Kühlaggregat und eine Entspannunsvorrichtung problemlos quantitativ ablassen.

Die am Beispiel einer Laborapparatur geschilderte typische technische Ausrüstung zur Durchführung des erfindungsgemäßen Verfahrens läßt sich ohne weiteres auf entsprechend größerdimensionierte Technikums- bzw. Produktionsapparaturen übertragen. Es lassen sich dazu im Prinzip alle technisch üblichen Rohr- bzw. Rohrbündelreaktoren verwenden.

Im Hinblick auf den Wasserstoffdruck wird das Verfahren in Stufe ii) vorzugsweise im Bereich von 200 bis 300 bar durchgeführt. Dabei sind Wasserstoffdrucke im Bereich von 220 bis 260 bar besonders bevorzugt.

Vorzugsweise wird die Hydrierung in Schritt ii) in Gegenwart eines Solvens durchgeführt. Insbesondere setzt man dabei alkoholische Verbindungen ein, insbesondere niedermolekulare primäre Alkanole wie Methanol und/oder Ethanol. Das Mengenverhältnis von Aldehyd und Solvens in Schritt ii) des erfindungsgemäßen Verfahrens ist an sich nicht kritisch, vorzugsweise arbeitet man jedoch mit Volumenverhältnissen Aldehyd zu Solvens im Bereich von 10:1 bis 1:10, wobei ein Bereich von 3:1 bis 1:1 besonders bevorzugt ist.

Die im Rahmen der vorliegenden Erfindung einzusetzenden Kupfer-Zink-Kontakte sind aus dem Stand der Technik bekannt. Sie werden gemäß **DE-A-42 42 466** hergestellt, indem man wäßrige Lösungen mit einem Gehalt an wasserlöslichen Kupfer-(II)- und Zink-(II)-Salze mit Alkalicarbonatverbindungen bis zu einem pH-Wert 6 bis 10 versetzt, den entstandenen Niederschlag abtrennt und trocknet, den getrockenen Katalysator bei Temperaturen von 400 bis 600 °C über einen Zeitraum von 1 bis 60 Minuten calciniert und anschließend den calcinierten Katalysator in stückige Form bringt. Im Hinblick auf weitere Einzelheiten der Herstellung der Kupfer-Zink-Kontakte sei ausdrücklich auf Seite 3, Zeilen 13 bis 34 der genannten DE-A-42 42 466 verwiesen.

Um den Anteil an einer weitergehenden Hydrierung des Zielproduktes (I) zu Nebenprodukten weitgehend zu vermeiden, hat es sich bei der kontinuierlichen Arbeitsweise in einem Festbettreaktor als günstig herausgestellt, den Volumenstrom des Aldehyds auf LHSV-Werte einzustellen, die vorzugsweise im Bereich von 0,3 bis 3,0 h⁻¹, insbesondere 0,6 bis 1,2 h⁻¹ liegen. Unter LHSV-Wert ("liquid hourly space velocity") ist dabei wie in der Literatur üblich der Volumenstrom der Flüssigkeit - bezogen auf das Volumen des festen Katalysators - zu verstehen. Die hier genannten LHSV-Werte sind allein auf den Aldehyd bezogen, d.h. das gegebenenfalls eingesetzte Solvens wird nicht berücksichtigt).

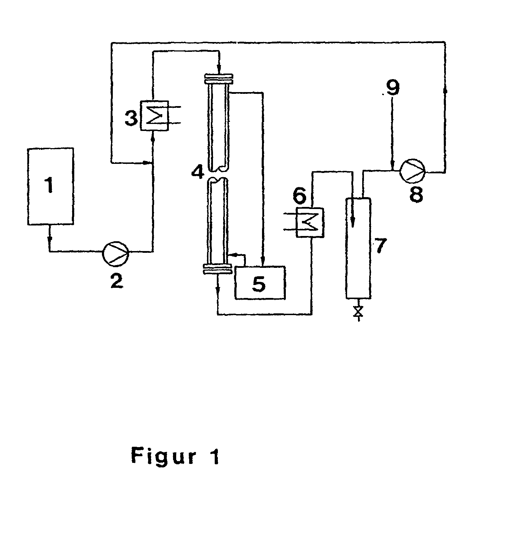

Die Kreisgasmenge wird auf Werte im Bereich von 1 bis 2 Dm³/h ("Druckkubikmeter pro Stunde"). eingestellt. Unter Kreisgasmenge ist zu verstehen: m³ Kreisgas pro Stunde bei einem Anlagendruck im Bereich von 250 bis 300 bar. Gemessen wird dieser Parameter im Druckbereich der eingesetzten Anlage (vergleiche hierzu Beispiel 3 der vorliegenden Anmeldung sowie die zugehörige Figur 1) nach der Gasumlaufpumpe mittels einer Turbine.

Der Volumenstrom wird im Rahmen dieser Erfindung üblicherweise auf GHSV-Werte im Bereich von 200-1000 h⁻¹, vorzugsweise 250 bis 500 h⁻¹ eingestellt. Unter GHSV-Wert ("gaseous hourly space velocity") ist dabei wie in der Literatur üblich der Volumenstrom des Trägergases - bezogen auf das Volumen des festen Katalysators - zu verstehen.

### Beispiele

### 1. Verwendete Substanzen

### 1.1. für die Aldolkondensation

alpha-Campholenaldehyd: 85%-ig (Fa. Glidco)
Propionaldehyd: 98%-ig (Fa. Riedel-de Häen)

KF auf Al₂O₃: 240 g basisches Aluminiumoxid wurden in 320 g einer 50%-igen wäßrigen Kaliumfluorid-Lösung suspendiert und anschließend am Rotationsverdampfer im Wasserstrahlvakuum zur Trockene eingeengt. Danach wurde der Katalysator noch 4 Stunden bei 130 °C und 50 mbar getrocknet.

### 1.2. für die Reduktion des ungesättigten Aldehyds

Kupfer-Zink-Katalysator: Der verwendete Kupfer-Zink-Katalysator wurde gemäß Beispiel A) der DE-A-42 42 466 unter Verwendung von Kupfer-(II)-Nitrattrihydrat, Zink-(II)-Nitrathexahydrat und Natriumcarbonat hergestellt.

### 2. Durchführung der Reaktionen

### 2.1. Aldolkondensation

### Beispiel 1

### Eingesetzte Mengen:

3,04 kg (20 mol) alpha-Campholenaldehyd
4,64 kg (80 mol) Propionaldehyd
400 g basisches Aluminiumoxid mit 40 % Kaliumfluorid beladen.

### Reaktion:

In einem 10-l-Glasreaktor wurden 1,5 kg eines Gemischs (Molverhältnis 1:4) Campholenaldehyd und Propionaldehyd vorgelegt. Unter Stickstoff (5 l/h), kräftigem Rühren und Kühlung wurden 400 g KF auf Al₂O₃ portionsweise zugegeben, wobei die Temperatur des Reaktionsgemisch zwischen 40 und 50 °C gehalten wurde. Anschließend wurden die restlichen 6,2 kg der Aldehydmischung kontinuierlich unter starkem Rühren und Stickstoff mit einer Dosierrate von 2 Liter pro Stunde zudosiert. Die Reaktion verlief exotherm und wurde durch Kühlen auf 40 °C gehalten. Nach der Zugabe wurde der Ansatz bei 50 °C über Nacht gerührt. Der Campholenaldehyd war danach vollständig abreagiert.

### Aufarbeitung:

Der Ansatz wurde zur Abtrennung des Katalysators in eine 2-l-Drucknutsche gepumpt und unter 5 bar Stickstoff filtriert. Der Filterkuchen wurden kurz mit 0,5 l Isopropanol gewaschen. Das Rohprodukt wurde wieder in den Reaktor überführt und bei Atmosphärendruck ca. 270 g organische Phase und 22 g Wasserphase bei Sumpftemperaturen bis zu 150 °C abdestilliert. Der im Reaktionsgefäß verbleibende Rückstand von 5,1 kg wurde zweimal mit je 2 l gesättigter Natriumsulfatlösung gewaschen. Der danach verbleibende Rückstand von 5 kg wurde als Rohprodukt weiter verarbeitet (der Gehalt an 2-Methyl-(2,2,3-trimethylcyclopent-3-en-1-yl)-but-2-en-1-al wurde gaschromatographisch zu 43 % bestimmt).

### Beispiel 2

### Eingesetzte Mengen:

3,04 kg (20 mol) alpha-Campholenaldehyd
2,9 kg (50 mol) Propionaldehyd
170 + 85 g (3 mol) Piperidin
120 + 60 g (3 mol) Eisessig
2 kg Toluol

### Reaktion:

In einem 10-1-Glasreaktor wurden 3,04 kg Campholenaldehyd und 2 kg Toluol vorgelegt und unter Rühren bei Raumtemperatur 2,9 kg Propionaldehyd, 170 g Piperidin und 120 g Eisessig hinzugefügt. Danach wurde das Gemisch an einem Wasserabscheider vier Stunden unter Rückfluß erhitzt und dabei 680 ml Reaktionswasser ausgekreist. Die gaschromatographische Analyse einer Probe des Reaktionsgemischs ergab einen Anteil von 15 % an nicht umgesetztem Edukt. Daher wurden weitere 85 g Piperidin und 60 g Eisessig zugegeben. Nach einer weiteren Stunde Erhitzen zum Rückfluß waren weitere 120 g Wasser ausgekreist und das Edukt vollständig umgesetzt.

### Aufarbeitung:

Nach Abdestillieren von 1,9 kg Toluol aus dem Reaktionsgemisch wurde zweimal mit je 2 1 Wasser gewaschen. Die organische Phase von 6,24 kg wurde an einer 30 cm Füllkörperkolonne destilliert (Siedepunkt 88 - 102 °C/0,1 mbar), wobei 3,27 kg eines gelblich gefärbten Produktes (gaschromatographisch bestimmte Reinheit: 85 %) erhalten wurden (85 % der Theorie).

### 2.2. Reduktion

### Beispiel 3 (erfindungsgemäß)

Anlage: Eingesetzt wurde ein mit einem Cu/Zn-Katalysator gefüllter ummantelter Hochdruckreaktor. Dieser war verbunden mit folgenden Aggregaten: Produktpresspumpe, Erhitzer, Kühler, Abscheider und Gasumlaufpumpe. Der Wärmeträgerkreislauf wurde mittels einer temperaturregelbaren Apparatur gesteuert. Der Aufbau der verwendeten Anlage ist in Figur 1 schematisch dargestellt.

### Bezüglich Figur 1 gilt folgende Bezugszeichenliste:

- 1: Edukt (Rohprodukt der Aldolkondensation gemäß Beispiel 2) / Methanol
- 2: Produktpreßpumpe
- 3: Erhitzer
- 4: Reaktor (ummantelt)
- 5: Wärmeträgeröl
- 6: Kühler
- 7: Abscheider
- 8: Gasumlaufpumpe
- 9: Wasserstoff-Zufuhr ("Frisch-H₂")

### Eingesetzte Mengen:

24,7 kg Rohprodukt der Aldolkondensation gemäß Beispiel 2 (= Edukt)
10,5 kg Methanol, technische Ware (= Lösungsmittel)
26 kg Cu-Zn-Katalysator

### Durchführung:

Nach Befüllen der Anlage mit dem Katalysator wurde der Reaktor geschlossen und die Anlage mit 250 bar H₂-Druck auf eventuelle Undichtigkeiten geprüft. Anschließend erfolgte die Aktivierung des Kontaktes durch kontinuierliche H₂-Dosierung bei einem Anlagendruck von 50 bar N₂-Druck und einer Temperaturerhöhung von 5 °C pro Stunde, wobei eine Endtemperatur von 200 °C eingestellt wurde. Hierbei wurde die Kreisgasmenge bei 1,5 Dm³/h gehalten. Nach dem Ende der Katalysator-Aktivierung - angezeigt durch einen H₂-Gehalt im Kreisgas von oberhalb 5 %, d.h. der Katalysator nimmt keinen weiteren Wasserstoff mehr auf - erfolgte der Gasaustausch in der Anlage.
Zur Durchführung des Hydrierungsprozesses wurden nach der vollständigen H₂-Entspannung 250 bar H₂ in die Anlage eingelassen und das Katalysatorbett durch Wasserstoff-, Reaktoreingangs- und Wärmeträgertemperatur auf 50 bis 55 °C eingestellt. Die Kreisgasmenge betrug 2 Dm³/h. Nach Erreichung der Konstanz der Betriebsparameter begann die Zudosierung eines Gemisches von 2 Volumenteilen des Rohproduktes der Aldolkondensation gemäß Beispiel 2 (= Edukt) und 1 Volumenteil Methanol (= Lösungsmittel), zunächst bei 10 l/h und sukzessiver Steigerung auf 40 l/h. Nach Zugabe der Gesamtmenge des Gemisches zeigte das Edukt-Wasserstoff-Gemisch eine Reaktionseingangs- und eine Wärmeträgerölausgangstemperatur von 55 °C. Hierbei betrug die Exothermie im Reaktor 5 bis 10 °C.
Das Hydrierprodukt und der Wasserstoff wurden nach Kühlung in einem Abscheider getrennt, der Wasserstoff zur Gasumlaufpumpe geleitet und das Endprodukt durch ein automatisches Entspannungssystem abgelassen.
Das auf diese Weise erhaltene Rohprodukt wurde anschließend gaschromatographisch charakterisiert. Es enthielt 86% des gewünschten Wertproduktes, 12% eines parfümistisch attraktiven Begleitproduktes sowie 1,8% an nicht umgesetztem Edukt. Nebenprodukte, wie sie beispielsweise bei höheren Temperaturen oder im Batchbetrieb mitentstehen, wurden praktisch nicht detektiert.

## Patentansprüche

1. Verfahren zur Herstellung von Alkyl-substituierten Butenolen der allgemeinen Formel (I)
R¹-CH₂-CH=CR²-CH₂OH (I)
worin R¹ eine gesättigte oder olefinisch ungesättigte Alkyl- oder Cycloalkylgruppe mit 4 bis 16 C-Atomen ist, die gegebenenfalls durch einen Alkyl-, Cycloalkyl-, Aryl- oder Alkarylrest substituiert sein kann - mit der Maßgabe, daß dieser Substituent maximal 12 C-Atome aufweist - und R² Wasserstoff oder eine Alkylgruppe mit 1 bis 6 C-Atomen bedeuten, durch Umsetzung von Aldehyden der Formel (II)
R¹-CH₂-CHO (II)
worin R¹ dieselbe Bedeutung hat wie in Formel (I), mit den entsprechenden niedrigen Aldehyden und anschließende Reduktion der dabei erhaltenen ungesättigten Aldehyde, wobei man
i) die Aldolkondensation in einem inerten organischen Lösungsmittel durchführt und
ii) die Reduktion der ungesättigten Aldehyde in Gegenwart eines gegebenenfalls calcinierten Kupfer-Zink-Kontaktes durchführt,
**dadurch gekennzeichnet, daß** man die Reduktion in Stufe ü) in kontinuierlicher und isothermer Fahrweise und bei Temperaturen im Bereich von 45 - 60 °C und bei einem Wasserstoffdruck im Bereich von 1 bis 300 bar kontinuierlich durchführt.

2. Verfahren nach Anspruch 1, wobei man die Aldolkondensation in einem unpolaren organischen Lösungsmittel durchführt, das mit Wasser ein Azeotrop bildet.

3. Verfahren nach Anspruch 1 oder 2, wobei man die Aldolkondensation in Gegenwart eines Ammoniumsalzes einer organischen Säure als Katalysator durchführt.

4. Verfahren nach einem der Ansprüche 1 bis 3, wobei R² in der Formel (I) eine Methylgruppe bedeutet.

5. Verfahren nach Anspruch 4, wobei man in Schritt i) Propionaldehyd in 2,5- bis 10-molarem Überschuß - bezogen auf den Aldehyd (II) - einsetzt.

6. Verfahren nach einem der Ansprüche 1 bis 5, wobei R¹ einen 4-(2,2,3-Trimethylcyclopent-3-en-1-yl)-rest bedeutet.

7. Verfahren nach einem der Ansprüche 1 bis 6, wobei man in Schritt i) ein organisches Lösungsmittel einsetzt, das ausgewählt ist aus der Gruppe Toluol, Xylol, Benzol, Cyclohexan und Methylcyclohexan.

## Claims

1. A process for producing alkyl-substituted butenols of the general formula (I),
R¹-CH₂-CH=CR²-CH₂OH (I)
wherein R¹ is a saturated or olefinically unsaturated alkyl or cycloalkyl group with 4 to 16 C atoms, which may optionally be substituted by an alkyl, cycloalkyl, aryl or alkaryl residue, with the proviso that this substituent comprises a maximum of 12 C atoms, and R² represents hydrogen or an alkyl group with 1 to 6 C atoms, by reacting aldehydes of the formula (II),
R¹-CH₂-CHO (II)
wherein R¹ represents the same as in formula (I), with the corresponding low aldehydes, and by the subsequent reduction of the unsaturated aldehydes thus obtained, wherein
i) the aldol condensation is carried out in an inert organic solvent, and
ii) the reduction of the unsaturated aldehydes is carried out in the presence of an optionally calcined copper-zinc contact,
**characterized in that** the reduction in step ii) is performed continuously in the continuous and isothermal mode of operation and at temperatures in the range of 45 to 60° C and at a hydrogen pressure in the range of 1 to 300 bar.

2. The process according to claim 1, wherein the aldol condensation is carried out in a non-polar organic solvent which forms an azeotrope with water.

3. The process according to claim 1 or 2, wherein the aldol condensation is carried out in the presence of an ammonium salt of an organic acid as a catalyst.

4. The process according to any of claims 1 to 3, wherein R² in formula (I) represents a methyl group.

5. The method according to claim 4, wherein propionaldehyde is used in step i) at a 2.5-10-fold molar excess based on the aldehyde (II).

6. The process according to any of claims 1 to 5, wherein R¹ represents a 4-(2,2,3-trimethyl cyclopent-3-en-1-yl) residue.

7. The process according to any of claims 1 to 6, wherein an organic solvent selected from the group consisting of toluene, xylene, benzene, cyclohexane and methylcyclohexane is used in step i).

## Revendications

1. Procédé de préparation de buténols substitués par un alkyle de formule générale (I)
R¹-CH₂-CH=CR²-CH₂OH (I)
dans laquelle R¹ est un groupe alkyle ou cycloalkyle saturé ou non saturé oléfiniquement, ayant de 4 à 16 atomes de carbone, qui peut être substitué le cas échéant par un reste alkyle, cycloalkyle, aryle ou alkylaryle,
avec la précision que ce substituant possède au maximum 12 atomes de carbone, et
R² signifie de l'hydrogène ou un groupe alkyle ayant de 1 à 6 atomes de carbone, par mise en réaction d'aldéhydes de formule (II),
R¹-CH₂-CHO (II)
dans laquelle R¹ a la même signification que dans la formule (I) avec les aldéhydes inférieurs correspondants et la réduction consécutive de l'aldéhyde non saturée obtenue de cette manière,
procédé dans lequel :
i) on effectue la condensation aldolique dans un solvant organique inerte, et
ii) on effectue la réduction de l'aldéhyde non saturé en présence d'un catalyseur par contact cuivre-zinc éventuellement calciné,
**caractérisé en ce qu'**
on effectue la réduction au stade ii) selon un processus en continu et isotherme et à des températures dans la plage de 45 à 60°C et à une pression d'hydrogène dans la zone de 1 à 300 bars, en continu.

2. Procédé selon la revendication 1,
dans lequel
on effectue la condensation aldolique dans un solvant organique non polaire qui forme avec l'eau un azéotrope.

3. Procédé selon l'une quelconque des revendications 1 ou 2,
dans lequel
on effectue la condensation aldolique en présence d'un sel d'ammonium d'un acide organique en tant que catalyseur.

4. Procédé selon l'une quelconque des revendications 1 à 3,
dans lequel
R² dans la formule (I) signifie un groupe méthyle.

5. Procédé selon la revendication 4,
dans lequel
on met en oeuvre à l'étape i) du propionaldéhyde en excès de 2,5 à 10 molaire rapporté à l'aldéhyde (II).

6. Procédé selon l'une quelconque des revendications 1 à 5,
dans lequel
R¹ signifie un reste 4-(2,2,3-triméthylcyclopent-3-en-1-yle).

7. Procédé selon l'une quelconque des revendications 1 à 6,
dans lequel
on met en oeuvre à l'étape i) un solvant organique qui est choisi dans le groupe du toluène, du xylène, du benzène, du cyclohexane et du méthylcyclohexane.
